(19) Europäisches Patentamt
European Patent Office
Office européen des brevets

(11) Publication number: **0 023 789 B1**

(12) # EUROPEAN PATENT SPECIFICATION

(45) Date of publication of patent specification: **25.01.84**

(51) Int. Cl.³: **C 08 B 37/08, C 08 J 9/28, C 08 L 5/08, C 12 N 11/10**

(21) Application number: **80302441.3**

(22) Date of filing: **18.07.80**

(54) **Process for preparing porous acylated chitin derivative and use of said derivative.**

(30) Priority: **20.07.79 JP 92898/79**

(43) Date of publication of application:
**11.02.81 Bulletin 81/6**

(45) Publication of the grant of the patent:
**25.01.84 Bulletin 84/4**

(84) Designated Contracting States:
**DE FR GB IT NL SE**

(56) References cited:
**EP - A - 0 013 181**
**GB - A - 565 343**
**US - A - 4 111 810**

**CHEMICAL ABSTRACTS, vol. 82, 1975 page 122, ref. 173048t Columbus, Ohio, US**

**PROCEEDINGS OF THE INT. CONFERENCE ON CHITIN/CHITOSAN,** ***held in*** **Boston May 1978 Massachusetts Inst. of Techn. Cambridge Sea Grant Program RICCARDO A.A. MUZZARELLI: "Modified chitosans and chromatographic performances", pages 335-347**

(73) Proprietor: **KUREHA KAGAKU KOGYO KABUSHIKI KAISHA**
**9-11 Horidome-cho 1-chome Nihonbashi Chuo-ku Tokyo (JP)**

(72) Inventor: **Koshugi, Junichi**
**No. 22 Daiichi-Kurehasoh**
**3-10-13 Nerima Nerima-ku, Tokyo (JP)**

(74) Representative: **Myerscough, Philip Boyd et al,**
**J.A.Kemp & Co. 14, South Square Gray's Inn**
**London, WC1R 5EU (GB)**

**EP 0 023 789 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for preparing porous acylated chitin derivative and use of said derivative

This invention relates to a process for producing an acylated chitin derivative by acylating a soluble derivative of chitin using an organic acid anhydride, and to its use.

Chitin is a mucopolysaccharide of poly-N-acetyl-D-glucosamine. The amount of it in nature compares favourably with that of cellulose. However, since chitin is a highly crystalline substance and its intermolecular bonding through aminoacetyl groups is extremely stable, it is very difficult to find an appropriate solvent to dissolve, disperse or swell chitin satisfactorily. Accordingly, development of the use of chitin resources is far behind that of cellulose and other polysaccharides.

A method for preparing a soluble derivative of chitin by carboxylalkylating the hydroxyl group of chitin and then by de-acetylating the thus-formed carboxylalkylated chitin is described in the Applicant's EPC Publication No. 0013512 (Application No. 79303066.9). Shaped materials of an acylated chitin derivative obtained by acylating this soluble chitin derivative with an organic acid anhydride are disclosed in the Applicant's EPC Publication No. 0013181 (Application No. 79303067.7). The shaped material that is obtained may have an outer surface comprising chemically stable chitin or N-acylated chitosan while its interior is made up of the amphoteric and polymeric electrolyte, de-N-acetylated carboxyalkylchitin. Particularly, the acylated shaped material is stable chemically and in living bodies, and is safe from the biological point of view. Moreover, it has cationic and anionic groups in its interior. Accordingly, its utilization, for instance, in blood perfusion and as an adsorbent which is orally administered to adsorb and remove toxins within the gastro-intestinal tracts is expexted.

However, the acylated shaped material has a small permeability to substances having a molecular weight of more than 70,000. It cannot effectively adsorb such substances or separate them. In other words, the shaped acylated chitin derivative is poor as a molecular sieve to molecules having molecular weight over a broad range and accordingly, it is unsatisfactory as an adsorbing and separating material for macromolecules such as protein, enzymes, etc. In addition, even if it were possible for acylated shaped material to adsorb and separate these macromolecules, it has been found that the material has insufficient strength.

EP—A2—0013181, published on 9th July 1980, forms prior art falling within the terms of EPC Article 54(3) in relation to the claims herein. Examples 12 and 13 of EP—A2—0013181 describe the acylation of specific chitin derivatives. An aqueous solution of a chitin derivative, to which ethylene glycol (Example 12) or ethylene glycol dimethyl ether (Example 13) had been added, is added to a solution of a suspending agent and an organic acid anhydride in toluene (Example 12) or xylene (Example 13) to affect acylation.

The present invention provides a process for preparing an acylated chitin derivative by acylating a soluble derivative of chitin using an organic acid anhydride by contacting an aqueous solution of the soluble derivative of chitin containing a diluent, an agent for regulating the porosity of the resultant porous acylated chitin derivative (hereinafter referred to as a porosity-regulating agent) and a surfactant (referred to herein as "the liquid mixture") with a liquid containing the organic acid anhydride and a surfactant so as to obtain a porous acylated chitin derivative. Further treatment may be carried out on the acylated product to cross-link it.

The acylated product can be satisfactorily employed as a molecular sieve and can have a uniform pore-size and excellent performance characteristics and strength. Pore-size can be adjusted depending on the manner in which the process is effected.

A soluble derivative of chitin for use in the invention is defined as a derivative of chitin which is soluble in water or an aqueous dilute acidic solvent, such as an aqueous dilute solution of acetic acid or hydrochloric acid. For instance, the soluble derivative of chitin can be a compound represented by the general formula (I):

$$[C_6H_8O_3\cdot(NH_2)_x\cdot(NHCOCH_3)_y\cdot(OR)_a\cdot(OH)_b]_n \qquad (I)$$

wherein R represents at least one group selected from a carboxyalkyl group with two to four carbon atoms, a hydroxyethyl group, a hydroxypropyl group, a dihydroxypropyl group or an alkyl group with one to three carbon atoms; $x$ is a number of from 0.1 to 1.0; $y = 1.0 - x$; $a$ is a number of from 0 to 1.0 and $b = 1.0 - a$, or a salt thereof. Salts include an alkali metal salt, an alkaline earth metal salt or an ammonium salt.

The de-N-acetylated products of carboxyalkyl chitin (including carboxymethyl chitin), hydroxyethyl chitin, hydroxypropyl chitin and dihydroxypropyl chitin of formula (I) have a degree of substitution (a) of preferably from 0.1 to 1.0, more preferably from 0.3 to 1.0, per pyranose ring and a degree of de-N-acylation (x) of from 0.1 to 1.0, preferably from 0.2 to 0.6 from the view point of their solubility in aqueous solution and the viscosity of such a solution. For the same reasons, the de-N-acylated products of alkylchitin (including methylchitin and ethylchitin) of formula (I) have a degree of substitution (a) of preferably from 0.1 to 1.0 and a degree of de-N-acylation preferably from 0.5 to 1.0, and further the de-

N-acylation product of chitin in which *a* is 0 in formula (I) has a degree of de-N-acetylation (x) of preferably from 0.5 to 1.0.

When the liquid mixture is contacted with the liquid containing the organic acid anhydride, the aqueous solution can be prepared by adding from 0.1 to 5 parts by weight of the diluent, from 0.001 to 1 parts by weight of the porosity-regulating agent and from 0.001 to 0.1 parts by weight of surfactant to one part by weight of an aqueous solution of the derivative of chitin. The order of addition and its method is not critical. Usually, first the diluent is added to the aqueous solution of the derivative of chitin to obtain an homogeneous solution, and then the porosity-regulating agent containing the surfactant is added to the homogeneous solution. This liquid mixture is usually in the state of an emulsified dispersion. However, it may take the appearance of a homogeneous solution depending upon its composition. The larger the concentration of the soluble derivative of chitin in the aqueous solution, the firmer and denser the material that is obtained. Generally, the concentration of the soluble chitin derivative is from 0.1 to 10% by weight.

The diluent should be compatible with the aqueous solution of the soluble derivative of chitin and the porosity-regulating agent. The diluent is used to adjust the viscosity of the liquid mixture and to assist the homogeneous dispersibility of the liquid mixture. When a material is prepared without using diluent, the pores in the material are irregular in size and the material is weak in strength.

As the diluent, alcohols with from one to four carbon atoms, pyridine or compounds represented by the general formula:

$$R{-}O{-}[(CH_2)_2O]_n{-}R'$$

wherein R and R′ independently represent a hydrogen atom or a $C_1$ to $C_4$ alkyl group and *n* is an integer of 1 to 3, or mixtures thereof, may be employed. The diluent may typically be methanol, ethanol, n-propanol, iso-propanol, pyridine, ethylene glycol, ethylene glycol monomethyl ether, ethylene glycol mono ethyl ether, ethylene glycol mono-n-butyl ether, ethylene glycol dimethyl ether, ethylene glycol diethyl ether, ethylene glycol di-n-butyl ether, diethylene glycol monomethyl ether, diethylene glycol monoethyl ether, diethylene glycol mono-n-butyl ether, diethylene glycol dimethyl ether, diethylene glycol diethyl ether and diethylene glycol di-n-butyl ether or mixtures of two or more of them.

The porosity-regulating agent is significant. It is used to obtain homogeneous acylation into within the acylated material that is formed and to make the thus-obtained material strong and highly porous. For these purposes, the porosity-regulating agent of the present invention should be insoluble in water, inert to the acylating agent and compatible with the acylating agent and the diluent. It can be benzene or a volatile alkylbenzene, such as toluene or xylene. The amount of porosity-regulting agent that is used may be determined depending upon the desired pore-size to pore-volume of the final product.

The surfactant is preferably non-ionic, for instance a monolaurate, monopalmitate, monostearate, monooleate or trioleate, of sorbitan or polyoxyethylene sorbitan. The surfactant is effective in dissolving or finely dispersing the diluent and the porosity-regulating agent in the aqueous solution of the soluble chitin derivative. It is further effective in dispersing fibrous material formed by diluting the soluble derivative of chitin with a large amount of the diluent.

The porous acylated chitin derivative can be easily obtained by treating the liquid mixture with a liquid containing the acylating agent and surfactant. Particularly by using an anhydride of an organic acid or a mixture of the anhydride and an organic acid as the acylating agent, the porous material can be extremely easily obtained in a short time period. The mode by which the liquid mixture is added to the acylating agent can be suitably selected according to the use and form of the final product.

The organic acid and the acid anhydride may be aliphatic and aromatic organic acid with from two to twenty carbon atoms and an anhydride thereof, respectively. For instance, acetic acid, propionic acid, butyric acid and valeric acid, and their anhydrides are suitable. These acids and acid anhydrides can be used singly or as a mixture of two or more of them. The organic acid anhydride may be used without dilution or after dilution with a solvent inert to the anhydride and, if used, the organic acid. Organic solvents such as benzene, toluene, xylene can be employed. The solvents can be used to adjust the rate of acylation and to make the after-treatment of the acylated product easy.

Acylation usually is carried out at a temperature of from 5 to 80°C, preferably from 5 to 60°C. However, in the case where the soluble derivative of chitin has a degree of de-N-acylation of over 0.6, a higher temperature is preferable. The acylated product is either chitin, O-acylated chitin, N-acylated chitosan, or a mixture thereof.

In one embodiment, the acylated porous product of the invention can be prepared by dropping the liquid mixture prepared as mentioned above into an organic acid anhydride containing surfactant under agitation to form dispersed small particles.

The amount of the organic acid anhydride used as the acylating agent is not particularly restricted. In general, from 1 to 100 equivalents, preferably from 5 to 20 equivalents, per equivalent of amino group of the de-N-acylated product of chitin or chitin derivative is employed. In order to regulate the dispersed state of the particles of the liquid mixture in the organic acid anhydride, it is preferably to

added from 10 to 1,000 times, preferably from 10 to 500 times, by weight of the liquid mixture of an organic solvent to the acylating agent.

The amount of surfactant added to the acylating agent is not particularly restricted. Usually it is employed in an amount of from 0.01 to 0.1 parts by weight per part by weight of the liquid mixture. A stirrer provided with usual stirring blades, static mixer or homogenizer may be used in dispersing and shaping the acylated final product. In the above-mentioned mode, reaction proceeds instantaneously from the surface of the droplets of the liquid mixture. An insoluble membrane made of acylated chitin derivative is formed on the surface of the droplet with the result that spherical capsule-like products are obtained. On continuation of the reaction, the organic acid anhydride diffuses into the droplet to form a gel which can be insolubilized. After carrying out the reaction for a predetermined time, gel-like porous spherical particles comprising the acylated chitin derivative are obtained by separating the particles and washing them to remove unreacted organic acid anhydride. The spherical particles may have a diameter ranging from 106 to $10^{-2}$m (1 to 10,000 microns) depending upon the degree of dispersing, the viscosity of the liquid mixture, etc.

In order to change the compactness of the gel-like porous material, the concentration of the soluble derivative of chitin in the aqueous solution, the temperature and the period of the reaction, the amount of the diluent, etc. can be changed. The compactness can be changed such that the threshold molecular weight of substances which can pass through the pores of the product of the present invention is in the range of from 500 to 400,000.

In addition, minute spherical particles of porous gel comprising the acylated chitin derivative can be prepared by first spraying an aqueous solution of the soluble derivative of chitin into a gaseous phase of the organic acid anhydride. The surface layers of droplets of the aqueous solution are instantly acylated to form an insoluble membrane. The droplets are then brought into contact with a solution containing the acylating agent.

The liquid mixture can be spun through nozzles into the liquid containing the organic acid anhydride. Fibers at least the surface layer of which is insolubilized, are obtained. These fibers are washed to obtained gel-like porous fibers. Alternatively, by using nozzles in the form of slits, gel-like porous films can be obtained. In the case of both films and fibers, it is also possible to obtain hollow material, and to control the thickness and the compactness of the membrane according to necessity as in the case of spherical particles. Other modes of adding the aqueous solution of the soluble derivative of chitin to the acylating solution can be employed.

The gel-like and porous shaped material prepared according to the present invention can have a broad water-content ranging from 10 to 100 times the weight of the material in a dry state, when obtained by the above methods.

The porous acylated material can be subjected to cross-linking. Cross-linking can be carried out, for instance, by the following method:

First, the water content of the gel-like porous acylated material is reduced to twice to 3 times the weight of the dried material by a treatment such as centrifugation. Then the gel-like material is immersed in an aqueous solution of sodium hydroxide (concentration of larger than 40% by weight) in an amount of more than twice, preferably from 4 to 30 times, the weight of the dry material. The immersion is carried out at a temperature lower than 15°C for from 1 to 5 hours. After the immersion is over, excess sodium hydroxide is removed such that the aqueous solution of sodium hydroxide remains in an amount of from 3 to 6 times the weight of the dry material. Then the mixture is left at a temperature of 10 to 0°C for one to 24 hours to obtain a material pre-treated with alkali. It is preferable next to freeze the immersed material at a temperature of from 0 to −30°C for one to 24 hours. This pre-treated material is then dispersed in water or an organic solvent containing a cross-linking agent in the amount of from 0.1 to 3 molar times, preferably 0.5 to 2 molar times, the molar amount of pyranose rings of the acylated product and is allowed to react for from 5 to 48 hours at a temperature lower than 15°C. After the reaction is over, the product is washed with water and is neutralized. A water-insoluble porous shaped material is obtained.

Suitable cross-linking agents are epoxy compounds such as epichlorohydrin, epibromohydrin, 2,3-dibromopropanol and 2,3-dichloropropanol. The degree of cross-linking may be from 0.01 to 0.3 per pyranose ring as determined by elementary analytical values.

It is easily possible to prepare shaped materials such as spherical particles, fibers and films in accordance with the present invention. The shaped material comprises an acylated product of a soluble derivative of chitin. The acylated product is porous, permeable, stable chemically in living bodies and safe, and can therefore by employed over a broad range of applications. Particularly spherical particles are utilizable as an ion-exchanger by leaving some amino groups intact by employing a controlled amount of acylating agent. The material can be used as a filler for a chromatographic column or as a base material on which an enzyme, antibody or antigen is to be immobilised.

Since the porous acylated material is extremely stable and safe to living bodies, it is utilizable for instance, in blood perfusion, as an adsorbent which can be orally administered to adsorb toxins within the gastro-intestinal tracts, or to coat an adsorbent. In addition, where the material is used in contact with blood, some amino groups can be left free to form polyions with an anti-thrombogenic substance such as heparin, chitosn sulfate and chitin sulfate, and, more preferably, the amino groups are sulfated.

The present invention is therefore effective in bringing about a greater utilization of chitin resources which have hitherto seen limited use.

The following Examples illustrate the present invention.

Example 1

$5 \times 10^{-3}$ Kg (5 g) of de-N-acylated product (degree of de-N-acylation of 0.9) of chitin were dissolved in $2 \times 10^{-1}$ Kg (200 g) of an aqueous 2% solution of acetic acid. $3 \times 10^{-3}$ Kg (3 g) of sorbitan monooleate as surfactant and $2 \times 10^{-2}$ Kg (20 g) of toluene as porosity-regulating agent were dissolved in $10^{-1}$ Kg (100 g) of ethanol as diluent. This solution was added to the above aqueous solution under agitation to emulsify the solution. $3 \times 10^{-2}$ Kg (30 g) of polyoxyethylene sorbitan monooleate and $5 \times 10^{-2}$ Kg (50 g) of acetic anhydride were dissolved in $3 \times 10^{-3}$ $m^3$ (3 litres) of toluene in a vessel provided with a bow-type stirrer, and then the above-mentioned emulsified liquid containing de-N-acylated chitin was added under agitation at 1,000 rpm at room temperature to make a dispersion in which reaction took place.

After reacting for one hour $2 \times 10^{-3}$ $m^3$ (2 litres) of ethanol was added to the dispersion under agitation to obtain a transparent spherically shaped material.

After separating the material by filtration and washing the separated material repeatedly with ethanol, the washed material was dispersed in distilled water and was neutralized by an aqueous 1N solution of sodium hydroxide. After filtration and repeated washing with water, gel-like porous particles of from $5 \times 10^{-5}$ to $1.5 \times 10^{-4}$ m (50 to 150 microns) diameter, when viewed under a microscope, were obtained. This product contained 20 Kg of water per Kg of dry matter. The elementary analytical composition of the dried product was as follows:

Carbon: 46.5%, Hydrogen: 6.8%,
Oxygen: 40.3% and Nitrogen: 6.7%

This data coincided well with those of chitin. In the infrared absorption spectrum of the dried specimen, no band appearing at 1500 to 1530 $cm^{-1}$ due to an amino group could be detected, suggesting the conversion of the amino group to an aminoacetyl group.

In addition, the dried specimen of the thus-obtained product did not dissolve in an aqueous 5% solution of acetic acid.

Example 2

$10^{-2}$ Kg (10 g) of a de-N-acylated product of hydroxyethyl chitin (degree of hydroxyethylation of 1.0 and degree of de-N-acetylation of 0.6) were dissolved in $1.5 \times 10^{-1}$ Kg (150 g) of an aqueous 2% solution of acetic acid. $3 \times 10^{-3}$ Kg (3 g) of polyoxyethylene sorbitan monooleate as surfactant and 10 g of xylene as porosity-regulating agent were dissolved in $5 \times 10^{-2}$ Kg (50 g) of ethyleneglycol monomethyl ether as diluent. This solution was added to the above-mentioned aqueous solution under agitation to make an emulsion. This emulsion was added to a solution prepared by dissolving $2.5 \times 10^{-2}$ Kg (25 g) of polyoxyethylene trioleate and $10^{-1}$ Kg (100 g) of propionic anhydride in $5 \times 10^{-3}$ $m^3$ (5 litres) of xylene within a Henschel mixer kept at a temperature of 20 to 25°C, under agitation of 5,000 rpm at room temperature to carry out reaction in a dispersed state.

After reacting for one hour $3 \times 10^{-3}$ $m^3$ (3 litres) of ethanol were added to the reaction mixture under agitation of 1,000 rpm to stop the reaction. The product was treated in the same way as in Example 1 to obtain a spherically shaped gel-like particles of a diameter of from $10^{-5}$ to $10^{-4}$ m (10 to 100 microns) when viewed under a microscope. The water content of the gel-like material was 10 Kg per Kg of dried matter. The elementary analytical composition of the dried matter was 50.5% of carbon, 6.7% of hydrogen, 36.4% of oxygen and 6.4% of nitrogen.

Example 3

$2 \times 10^{-2}$ Kg (20 g) of the sodium salt of de-N-acetylated carboxymethylchitin (degree of carboxymethylation of 0.5 and degree of de-N-acylation of 0.25) were dissolved in $1.8 \times 10^{-1}$ Kg (180 g) of water. $2 \times 10^{-3}$ Kg (2 g) of polyoxyethylene trioleate as surfactant and $10^{-2}$ Kg (10 g) of toluene as porosity-regulating agent were dissolved in a mixed solvent consisting of $2 \times 10^{-2}$ Kg (20 g) of ethylene glycol dimethyl ether and $10^{-2}$ Kg (10 g) of pyridine as diluents. This solution was added to the above-mentioned aqueous solution under agitation, and the whole mixture was subjected to de-airing to be the starting material for spinning.

Fibers of acylated chitin derivative were obtained by spinning the starting material using a spinning apparatus which was provided with a tank for the starting material, a reacting vessel $5 \times 10^{-2}$ m (50 mm) in diameter and 5 m (5,000 mm) in length provided with 25 nozzle-holes $5 \times 10^{-5}$ (0.05 mm) in diameter, a roller, a solvent-removing vessel containing a mixture of ethanol and water, and another solvent-removing vessel containing hot water at a temperature of 100°C and a take-up winder. The composition of the solution introduced into the reacting vessel kept at a temperature of 50°C was as follows:

toluene : acetic anhydride : polyoxyethylene monooleate = 10 : 5 : 1 (by weight). The spinning was carried out at an output pressure of $10^4$ $Kg/m^2$ (1 $kg/cm^2$).

The thus obtained fiber had a diameter of $1.5 \times 10^{-5}$ m (15 microns) in the dry state and $5 \times 10^{-5}$ m (50 microns) after immersion in water.

Example 4

The gel-like product obtained in Example 2 was dehydrated by centrifugation such that its water-content was $2 \times 10^{-3}$ $m^3$/Kg (2 ml per gram) of dry product. The thus-dehydrated gel was immersed in 23 times its weight of an aqueous 43.5% solution of sodium hydroxide. After compressing the thus-immersed gel to remove the aqueous alkali solution such that the water content of the gell was 3 times the weight of the dry product and leaving it to stand at 0°C for 2 hours, the gel was frozen at a temperature of −20°C for one hour.

A solution of epichlorhydrin, corresponding to twice the molar amount of the above-mentioned gel-like product when dry in an amount of isopropyl alcohol corresponding to 50 times the weight of the gel-like product when dry was introduced into a flask provided with a stirrer and kept at a temperature of 0 to 5°C. The above-mentioned frozen gel-like product was added to this solution to carry out reaction for 5 hours. The reaction was carried out at a temperature of 15°C for a further 5 hours. After the reaction was over, the reaction mixture was filtered to obtain a solid material which was washed with ethanol and then was dispersed in distilled water, and was neutralized with an aqueous 1N solution of hydrochloric acid while cooling from outside. The thus-obtained solid material was separated by filtration and washed with water to obtained spherical particles of gel-like material.

It was found from the results of infrared absorption spectroscopy that the product had not been de-acetylated. The degree of cross-linking in the produce was 0.10 per pyranose ring.

Example 5

The spherically shaped cross-linked gel-like product prepared in Example 4 was sifted to a size of from $5 \times 10^{-5}$ to $10^{-4}$ m (50 to 100 microns). The sifted particles filled a column $2 \times 10^{-2}$ m (2 cm) in inner diameter to a volume of $1.5 \times 10^{-4}$ $m^3$ (150 ml).

A solution prepared by dissolving $2 \times 10^{-5}$ Kg (20 mg) of each of blue-dextran, dextran of molecular weight of $20^4$, dextran of molecular weight of $10^5$ and dextran of molecular weight of $2.5 \times 10^5$ together in $2 \times 10^{-6}$ $m^3$ (2 ml) of distilled water was poured into the column. The contents of the column were eluted with distilled water at a rate of $1.66 \times 10^{-8}$ $m^3$/s (1 ml/min).The eluants were analyzed by a total carbon analyzer and an infrared analyzer. The elution-pattern thus obtained is shown in Table 1. As is clearly seen in Table 1, the gel-like particles were capable of separating substances of molecular weight of $2.5 \times 10^5$.

TABLE 1
Elution Pattern

| Components of specimen | Amount of Eluant: $m^3 \times 10^{-6}$ (ml) |
|---|---|
| Blue-dextran of mol. wt. $2 \times 10^6$ | 55 to 65 |
| Dextran of mol. wt. of $2.5 \times 10^5$ | 75 to 85 |
| Dextran of mol. wt. of $1.0 \times 10^5$ | 100 to 110 |
| Dextran of mol. wt. of $1.0 \times 10^4$ | 120 to 130 |

Example 6

A homogeneous solution was prepared by dissolving $5 \times 10^{-3}$ Kg (5 g) of a de-N-acetylated product of glyceride-chitin (degree of substitution of 0.5 and degree of de-acetylation of 0.5) in $10^{-1}$ kg (100 g) of an aqueous 5% solution of acetic acid containing also $2 \times 10^{-5}$ kg (20 mg) of blue-dextran of molecular weight $2 \times 10^6$. Into this solution a liquid prepared by adding $10^{-3}$ Kg (1 g) of polyoxyethylene sorbitan monoleate as surfactant and $5 \times 10^{-3}$ Kg (5 g) of benzene as porosity-regulating agent to $10^{-1}$ kg (100 g) pyridine as diluent was slowly added to make a homogeneous liquid, hereinafter referred to as liquid *A*.

In a flask provided with a stirrer, $2 \times 10^{-3}$ $m^3$ (2 litres) of toluene, $5 \times 10^{-5}$ $m^3$ (50 ml) of acetic anhydride and $10^{-2}$ Kg (10 g) of polyoxyethylene sorbitan monooleate were introduced under agitation to form a homogeneous solution. Liquid *A* was added to this solution under agitation to carry out reaction for 2 hours at room temperature under agitation of 1,000 rpm.

On carrying out the same procedures to the reaction mixture as in Example 1, a gel-like product

light blue in colour was obtained consisting of minute spherical particles of $5 \times 10^{-5}$ to $2 \times 10^{-4}$ m (50 to 200 microns) in diameter, when viewed under a microscope, with a clearly porous surface. The product did not dissolve either in acids or in alkaline solutions. The light blue colour of the product did not disappear when it was put into acids and alkalies. From this finding, it is considered that the high molecular weight blue dextran was confined in the gel-like particles.

Example 7

Two types of gel-like porous particles were prepared as in Example 1 except that $10^{-2}$ and $2.5 \times 10^{-2}$ kg (10 and 25 g), respectively, of toluene were employed instead of $2 \times 10^{-2}$ kg (20 g) of toluene as porosity-regulating agent.

The state of the surfaces of the thus-obtained porous particles were taken by scanning electron microscopic photography and are shown in Figs. 1, 2 and 3. Fig. 1 shows a photograph magnified by 2,800 times when the amount of toluene used was $10^{-2}$ kg (10 g), Fig. 2 shows the same, however, magnified by 1,400 times, and Fig. 3 shows a photograph magnified by 1,400 times of the product when $2.5 \times 10^{-2}$ kg (25 g) of toluene was used. For the purpose of comparison, the state of surface of the spherically shaped material prepared in Example 1 in which no toluene as the porosity-regulating agents was used is shown in Fig. 4. These microphotographs of the images of the scanning electron microscope were taken after substituting the water of the gel-like particles with isoamyl acetate and drying at its critical temperature.

As are clearly seen in Figs. 1, 2 and 3, the pore-size and their distribution in the shaped material can be regulated by selecting the amount of porosity-regulating agent, toluene, in the aqueous solution of the soluble derivative of chitin.

**Claims**

1. A process for producing an acylated chitin derivative by acylating a soluble derivative of chitin using an organic acid anhydride characterised in that an aqueous solution of the soluble derivative of chitin containing a diluent, a porosity-regulating agent and a surfactant is contacted with a liquid containing the organic acid anhydride and a surfactant so as to obtained a porous acylated chitin derivative.

2. A process according to claim 1 wherein said soluble derivative of chitin is a compound represented by the general formula (I):

$$[C_6H_8O_3 \cdot (NH_2)_x \cdot (NHCOCH_3)_y \cdot (OR)_a \cdot (OH)_b]_n \qquad (I)$$

wherein (i) R represents a carboxymethyl group, a hydroxyethyl group or a dihydroxypropyl group and *a* is from 0.1 to 1.0 and *x* is from 0.1 to 1.0, or (ii) R represents a methyl or ethyl group and *a* is from 0.1 to 1.0 and *x* is from 0.5 to 1.0, or (iii) *a* is 0 and *x* is from 0.5 to 1.0; and $y = 1.0 - x$ and $b = 1.0 - a$.

3. A process according to claim 1 or 2 wherein said diluent is an alcohol with one to four carbon atoms, pyridine, an ether represented by the general formula:

$$R{-}O{-}[(CH_2)_2O]_n{-}R'$$

wherein R and R′ independently represent a hydrogen atom or an alkyl group with one to four carbon atoms and *n* represents an integer of 1, 2 or 3, or a mixture thereof.

4. A process according to any one of the preceding claims wherein said porosity-regulating agent is benzene, toluene or xylene.

5. A process according to any one of the preceding claims, wherein said surfactant is a monolaurate, monopalmitate, monostearate, monoolerate or trioleate of sorbitan or of polyoxyethylene sorbitan.

6. A process according to any one of the preceding claims wherein said aqueous solution contains from 0.1 to 5 parts by weight of said diluent, from 0.001 to 1 parts by weight of said porosity-regulating agent and from 0.001 to 0.1 parts by weight of surfactant per part by weight of said aqueous solution.

7. A process according to any one of the preceding claims, wherein the concentration of said soluble derivative of chitin in said aqueous solution is from 0.1 to 10% by weight.

8. A process according to any one of the preceding claims wherein the concentration of surfactant in said liquid containing said organic acid anhydride is from 0.01 to 0.1% by weight.

9. A process according to any one of the preceding claims characterised in that the porous acylated chitin derivative is cross-linked.

10. An immobilised enzyme wherein an enzyme is immobilised on a base material which is an acylated chitin derivative which has been prepared by a process as claimed in any one of the preceding claims.

11. An adsorbent coated with an acylated chitin derivative which has been prepared by a process as claimed in any one of claims 1 to 9.

12. A polyion of an acylated chitin derivative which has been prepared by a process as claimed in any one of claims 1 to 9 with an anti-thrombogenic substance.

**Revendications**

1. Procédé de production d'un dérivé de chitine acylé par acylation d'un dérivé soluble de chitine en utilisant un anhydride d'acide organique, caractérisé en ce qu'une solution aqueuse du dérivé soluble de chitine contenant un diluant, un agent régulateur de porosité et un agent tensio-actif est mise en contact avec un liquide contenant l'anhydride d'acide organique et un agent tensio-actif de façon à obtenir un dérivé poreux de chitine acylée.

2. Procédé selon la revendication 1, caractérisé en ce que le dérivé soluble de chitine est un composé représenté par la formule générale (I):

$$[C_6H_8O_3\cdot(NH_2)_x\cdot(NHCOCH_3)_y\cdot(OR)_a\cdot(OH)_b]_n \quad (I)$$

dans laquelle:

(i) R représente un groupe carboxyméthyle, un groupe hydroxyéthyle ou un groupe dihydroxypropyle et *a* varie de 0,1 à 1,0 et *x* varie de 0,1 à 1,0 ou

(ii) R représente un groupe méthyle ou éthyle et *a* varie de 0,1 à 1,0 et *x* varie de 0,5 à 1,0 ou

(iii) *a* vaut 0 et *x* varie de 0,5 à 1,0 et $y = 1,0 - x$ et $b = 1,0 - a$.

3. Procédé selon l'une quelconque des revendications 1 ou 2, caractérisé en ce que ledit diluant est un alcool ayant de 1 à 4 atomes de carbone, la pyridine, un éther représenté par la formule générale:

$$R{-}O[(CH_2)_2O]_nR'$$

dans laquelle R et R' représentent indépendamment l'un de l'autre un atome d'hydrogène ou un groupe alcoyle ayant de 1 à 4 atomes de carbone et *n* représente un nombre entier de 1, 2 ou 3 ou un mélange de ceux-ci.

4. Procédé selon l'une quelconque des revendications précédentes, caractérisé en ce que l'agent régulant la porosité est le benzène, le toluène ou le xylène.

5. Procédé selon l'une quelconque des revendications précédentes, dans lequel l'agent tensio-actif est un monolaurate, monopalmitate, monostéarate, monooléate ou trioléate de sorbitan ou de sorbitan de polyoxyéthylène.

6. Procédé selon l'une quelconque des revendications précédentes, caractérisé en ce que la solution aqueuse contient de 0,1 à 5 parties en poids du diluant, de 0,001 à 1 parties en poids dudit agent régulant la porosité et de 0,001 à 0,1 partie en poids d'agent tensio-actif par partie en poids de ladite solution aqueuse.

7. Procédé selon l'une quelconque des revendications précédentes, caractérisé en ce que la concentration dudit dérivé soluble de chitine dans ladite solution aqueuse est de 0,1 à 10% en poids.

8. Procédé selon l'une quelconque des revendications précédentes, dans laquelle la concentration du tensio-actif dans ledit liquide contenant ledit anhydride d'acide organique varie de 0,01 à 0,1% en poids.

9. Procédé selon l'une quelconque des revendications précédentes, caractérisé en ce que le dérivé poreux de chitine acylée est réticulé.

10. Enzyme immobilisée, caractérisée en ce qu'une enzyme est immobilisée sur un matériau de base qui est un dérivé de chitine acylée, lequel a été préparé par un procédé selon l'une quelconque des revendications précédentes.

11. Adsorbent revêtu d'un dérivé de chitine acylée préparé par un procédé selon l'une quelconque des revendications 1 à 9.

12. Polyion d'un dérivé de chitine acylée qui a été préparé par le procédé selon l'une quelconque des revendications 1 à 9 avec une substance anti-thrombogénique.

**Patentansprüche**

1. Verfahren zur Herstellung eines acylierten Chitin-derivats durch Acylierung eines löslichen Derivats des Chitins unter Verwendung eines organischen Säureanhydrids, dadurch gekennzeichnet, daß eine wässrige Lösung des löslichen Derivats des Chitins, die ein Verdünnungsmittel, ein Mittel zur Porositätsregulierung sowie ein Tensid enthält, mit einer Flüssigkeit in Kontakt gebracht wird, die das organischen Säureanhydrid sowie ein Tensid enthält, um ein poröses acyliertes Chitinderivat zu erhalten.

2. Verfahren nach Anspruch 1, bei dem das genannte lösliche Derivat des Chitins eine Verbindung ist, die durch die allgemeine Formel (I) wiedergegeben wird:

$$[C_6H_8O_3 \cdot (NH_2)_x \cdot (NHCOCH_3)_y \cdot (OR)_a \cdot (OH)_b]_n \qquad (I)$$

in der (i) R eine Carboxymethylgruppe, eine Hydroxyethylgruppe oder eine Dihydroxypropylgruppe bedeutet und a von 0,1 bis 1,0 und x 0,1 bis 1,0 ist, oder (ii) R eine Methyl- oder Ethylgruppe bedeutet und a von 0,1 bis 1,0 und x 0,5 bis 1,0 ist, oder (iii) a ist 0 und x ist von 0,5 bis 1,0; und y ist 1,0 — x und b = 1,0 — a.

3. Verfahren nach Anspruch 1 oder 2, bei dem das genannte Verdünnungsmittel ein Alkohol mit einem bis vier Kohlenstoffatomen, Pyridin, ein Äther der allgemeinen Formel:

$$R{-}O{+}(CH_2)_2O{+}_nR'$$

ist, in der R und R' unabhängig voneinander ein Wasserstoffatom oder eine Alkylgruppe mit einem bis vier Kohlenstoffatomen sind und n eine der ganzen Zahlen 1, 2 oder 3 ist, oder eine Mischung dieser Substanzen.

4. Verfahren nach einem beliebigen der vorangehenden Ansprüche, bei dem das genannte Mittel zur Porositätsregulierung Benzol oder Toluol oder Xylol ist.

5. Verfahren nach einem beliebigen der vorangehenden Ansprüche, bei dem das genannte Tensid ein Monolaurat, Monopalmitat, Monostearat, Monooleat oder Trioleat von Sorbitan oder Polyoxyethylensorbitan ist.

6. Verfahren nach einem beliebigen der vorangehenden Ansprüche, bei dem die genannte wässrige Lösung von 0,1 bis 5 Gew.-Teile des genannten Verdünnungsmittels, von 0,001 bis 1 Gew.-Teile des genannten Mittels zur Regulierung der Porosität und von 0,001 bis 0,1 Gew.-Teile des Tensids pro Gew.-Teil der genannten wässrigen Lösung enthält.

7. Verfahren nach einem beliebigen der vorangehenden Ansprüche, bei dem die Konzentration des genannten löslichen Chitinderivats in der genannten wässrigen Lösung von 0,1 bis 10 Gew.-% beträgt.

8. Verfahren nach einem beliebigen der vorangehenden Ansprüche, bei dem die Konzentration des Tensids in der genannten Flüssigkeit, die das genannte organische Säureanhydrid enthält, von 0,01 bis 0,1 Gew.-% beträgt.

9. Verfahren nach einem beliebigen der vorangehenden Ansprüche, dadurch gekennzeichnet, daß das poröse acylierte Chitinderivat vernetzt ist.

10. Ein immobilisiertes Enzym, bei dem ein Enzym auf einem Trägermaterial immobilisiert ist, das ein acyliertes Chitinderivat ist, das nach einem Verfahren gemäß einem beliebigen der vorangehenden Ansprüche hergestellt wurde.

11. Ein Adsorbens, das mit einem acylierten Chitinderivat überzogen ist, das nach einem Verfahren nach einem beliebigen der Ansprüche 1 bis 9 hergestellt wurde.

12. Ein Polyion eines acylierten Chitinderivats, das nach einem Verfahren nach einem beliebigen der Ansprüche 1 bis 9 hergestellt wurde, mit einer anti-thrombogenen Substanz.

Fig. 1

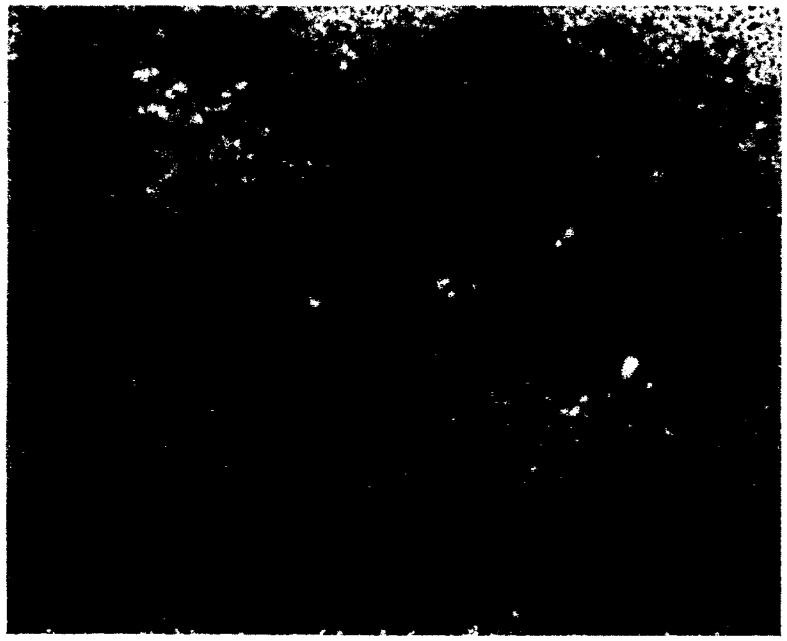

X2800

Fig. 2

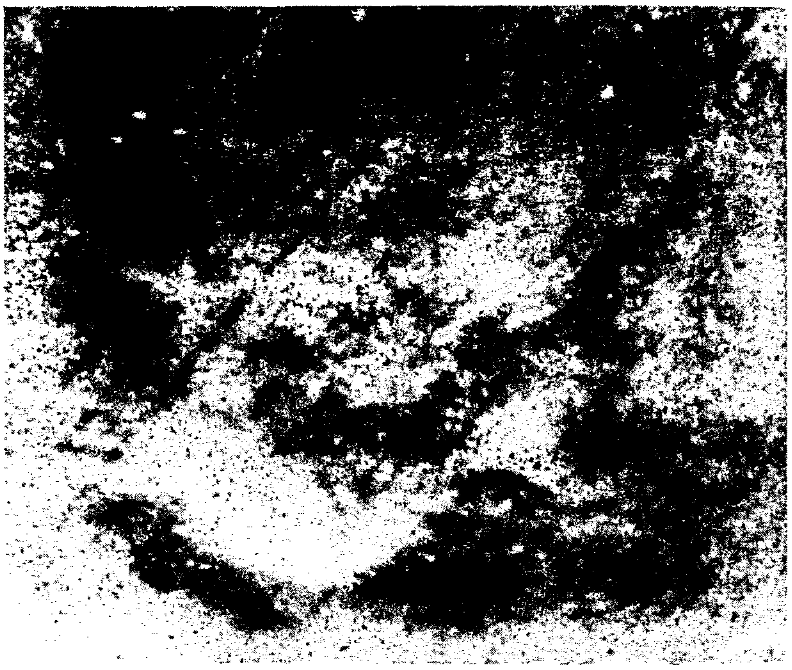

X1400

Fig. 3

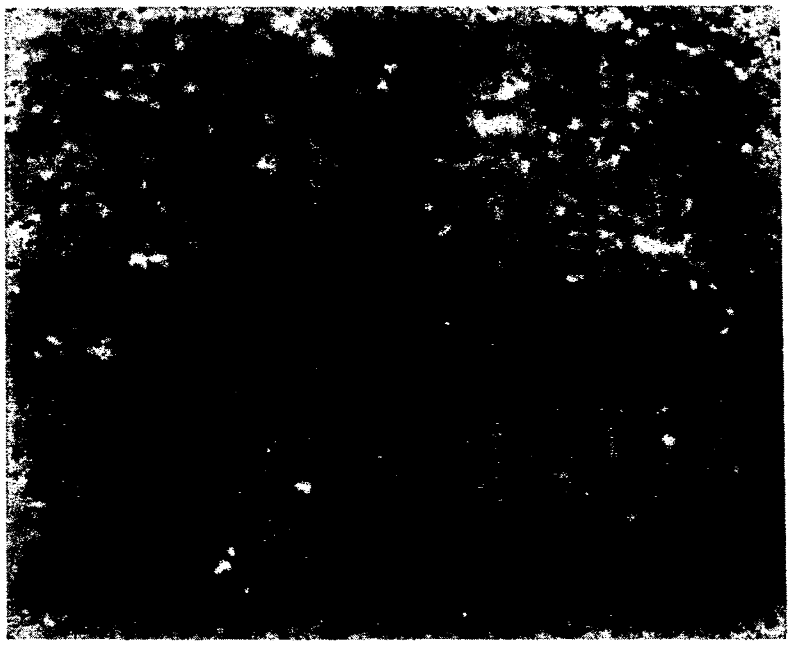

X1400

Fig. 4

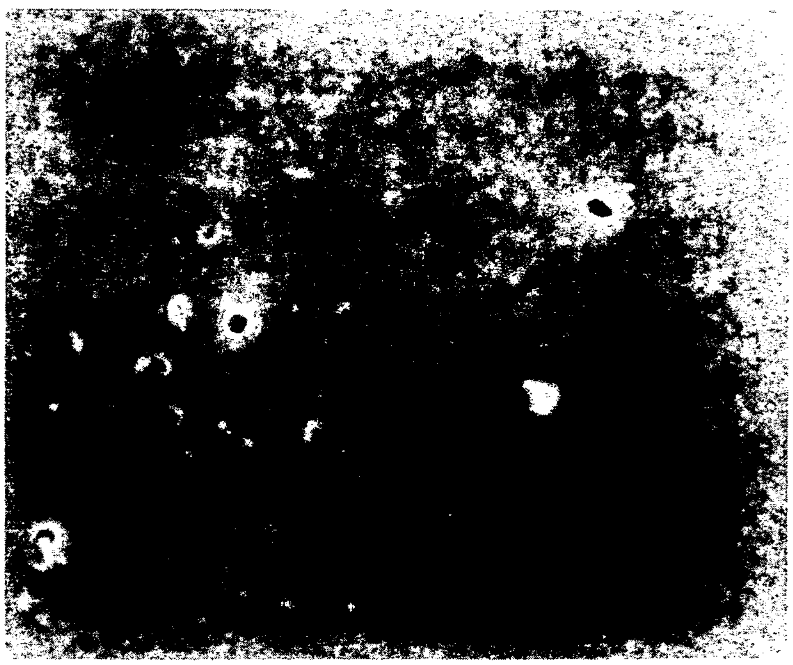

X3000